# DEMANDE DE BREVET EUROPEEN

(11) **EP 1 433 458 A1**
(43) Date de publication de la demande: **30.06.2004**
(21) Numéro de dépôt: 03293094.3
(22) Date de dépôt: 10.12.2003
(51) Int. Cl.: A61K 7/02, A61K 7/48

(54) **Composition cosmétique exempte de lanoline contenant un ester aromatique d'acide hydroxylé**

(30) Priorité: 23.12.2002 FR 0216533
(71) Demandeur: L'OREAL, 75008 Paris (FR)
(72) Inventeur: Filippi, Vanina, 75015 Paris (FR); Salem, Sophie, 94800 Villejuif (FR); Auguste, Frédéric, 94550 Chevilly-Larue (FR)
(74) Mandataire: Boulard, Denis

(57) **Abrégé**

L'invention se rapporte à une composition cosmétique de soin ou de maquillage, exempte de lanoline ou de dérivés de lanoline, contenant au moins un ester liquide à température ambiante, résultant de l'estérification, par un acide aromatique, d'au moins un groupe hydroxyle pendant ou en bout de chaîne d'un composé hydroxylé aliphatique. Cette composition contient un composé pâteux différent de la lanoline ou de l'un de ses dérivés et permet d'obtenir un dépôt sur les matières kératiniques glissant, brillant et confortable.

Le composé hydroxylé aliphatique est en particulier un acide gras hydroxylé tel que l'acide ricinoléique ou l'acide hydroxy stéarique ou un de ses esters.

La composition contient avantageusement un composé pâteux ayant une dureté à 25°C comprise entre 0,001 à 0,5 MPa, de préférence de 0,002 à 0,4 MPa, et dont la fraction liquide à 23°C est comprise entre 9 et 97% en poids.

## Description

La présente invention se rapporte à une composition cosmétique de maquillage ou de soin de la peau, y compris du cuir chevelu, aussi bien du visage que du corps humain, des lèvres ou des phanères des êtres humains, comme les cheveux, les cils, les sourcils ou les ongles, comprenant un milieu cosmétiquement acceptable contenant un ester aliphatique d'ester particulier. Cette composition est exempte de lanoline et elle contient un ou plusieurs esters issus de l'estérification totale ou partielle d'un composé aliphatique hydroxylé avec un acide aromatique. Cette composition possède des propriétés cosmétiques remarquables et confère en particulier au maquillage ou au soin des propriétés de brillance, de glissant à l'application, et de confort et de non collant.

La composition de l'invention peut en particulier constituer un produit de maquillage du corps, des lèvres ou des phanères d'êtres humains ayant en particulier des propriétés de soin et/ou de traitement non thérapeutique. Elle constitue notamment un rouge à lèvres ou un brillant à lèvres, un fard à joues ou à paupières, un produit pour tatouage, un mascara, un eye-liner, un vernis à ongles, un produit de bronzage artificiel de la peau, un produit de coloration ou de soin des cheveux.

Il existe de nombreuses compositions cosmétiques pour lesquelles les propriétés de brillance du film déposé, après application sur les matières kératiniques (peau, lèvres, phanères), sont souhaitables. On peut citer par exemple les rouges à lèvres, les vernis à ongles ou encore certains produits capillaires. Dans cette optique, le formulateur dispose de plusieurs types de matières premières et notamment des lanolines utilisées en association avec des huiles dites « brillantes », comme des polymères huileux tels que a) les polybutènes qui ont une viscosité élevée (typiquement supérieure à 2 819 Pa.s à 23°C mesurée avec un viscosimètre Brookfield RV équipé d'une aiguille n°1 tournant à 0,5 tr/min), b) des esters d'acide ou d'alcool gras dont le nombre de carbone est élevé (typiquement supérieur à 16), ou encore c) certaines huiles végétales.

Les esters résultants de l'estérification partielle ou totale d'un composé aliphatique hydroxylé avec un acide aromatique, comme décrit dans la demande de brevet EP 1 097 699, confèrent aux produits cosmétiques de bonnes propriétés d'application, de brillance et de dispersion des pigments. Les formules décrites dans ce document contiennent toutes de la lanoline pour apporter à ces compositions de l'onctuosité et du confort. Les lanolines présentent cependant l'inconvénient d'être sensibles à la chaleur et aux ultraviolets, et ont tendance à s'oxyder dans le temps avec un dégagement d'odeur désagréable, ce qui limite leur utilisation dans les compositions cosmétiques. De plus, lorsque les lanolines sont associées avec des huiles couramment utilisées dans le domaine cosmétique, les compositions obtenues présentent des problèmes de collant, qui sont d'autant plus prononcés que l'huile utilisée possède une viscosité élevée.

L'invention a justement pour objet une composition cosmétique de soin ou de maquillage des matières kératiniques et notamment de la peau et/ou des lèvres et/ou des phanères permettant de remédier aux inconvénients cités ci-dessus.

De façon surprenante, les inventeurs ont trouvé qu'il était possible d'obtenir une composition contenant un ou plusieurs esters issus de l'estérification totale ou partielle d'un composé aliphatique hydroxylé avec un acide aromatique, qui est brillante, confortable et non collante et qui ne contient pas de lanoline.

Cette composition présente en outre une bonne dispersion des pigments et/ou des charges présents dans la composition, elle n'exsude pas lorsqu'elle est sous forme de stick, elle présente de bonnes propriétés d'étalement et de glissant et confère, en outre, au film déposé des propriétés de brillance, de confort, de bonne tenue dans le temps (non virage de couleur pendant au moins 3 heures, disparition du maquillage de façon homogène), de non collant et de non gras. Elle est, en outre, stable notamment plusieurs mois à température ambiante (25°C pendant plus d'un an), mais aussi à la chaleur (47°C pendant 2 mois) et aux ultraviolets, sans dégradation de l'odeur au cours du temps.

De façon plus précise, l'invention a pour objet une composition cosmétique de soin ou de maquillage des matières kératiniques, contenant au moins un ester à groupement aromatique, liquide à température ambiante, résultant de l'estérification par un acide aromatique d'au moins un groupe hydroxyle pendant ou en bout de chaîne d'un composé aliphatique hydroxylé choisi parmi les acides aliphatiques hydroxylés et leurs esters, ladite composition étant exempte de lanoline ou de dérivés de lanoline.

On entend par dérivés de lanoline, notamment la lanoline oxypropylénée, la lanoline acétylée ou le lanolate d'isopropyle, par exemple la cire de lanoline oxypropylénée (5 OP) commercialisée sous la référence EMERY 1695 par COGNIS.

La composition selon l'invention contient avantageusement au moins un composé pâteux différent de la lanoline ou de ses dérivés.

### Ester à groupement aromatique

La composition comprend au moins un ester à groupement aromatique, liquide à température ambiante, résultant de l'estérification par un acide aromatique d'au moins un groupe hydroxyle pendant ou en bout de chaîne d'un composé aliphatique hydroxylé choisi parmi les acides aliphatiques hydroxylés et leurs esters.

Par composé aliphatique hydroxylé, on entend un acide hydroxy-carboxylique aliphatique ou un ester d'acide hydroxy-carboxylique aliphatique. L'acide (non estérifié) comporte notamment de 2 à 40 atomes de carbone, de préférence de 10 à 34 atomes de carbone et mieux de 12 à 28 atomes de carbone ; il comporte, en outre, de 1 à 20 groupes hydroxyle, de préférence de 1 à 10 groupes hydroxyle et mieux de 1 à 6 groupes hydroxyle, susceptible(s) d'être estérifié(s) par l'acide aromatique. Le composé hydroxylé sous forme d'ester résulte de l'estérification de la fonction -COOH d'un acide aliphatique hydroxy-carboxylique par un alcool aliphatique qui peut comprendre de 1 à 40 atomes de carbone et mieux de 3 à 30 atomes de carbone. Cet alcool peut être un monoalcool ou un polyol. Les esters issus de la réaction de l'acide hydroxy-carboxylique aliphatique avec un polyol peuvent être des esters partiellement ou totalement estérifiés.

De préférence le composé aliphatique hydroxylé est choisi parmi les esters issus d'acide hydroxy-carboxylique aliphatique. Autrement dit, l'ester aromatique liquide de la composition de l'invention est un ester d'ester. Avantageusement, cet ester aromatique est un ester d'ester d'acide gras dont le reste d'acide gras comporte au moins 12 atomes de carbone. De préférence, le groupe hydroxyle engagé dans l'estérification par l'acide aromatique est porté sur la partie acide du composé hydroxylé.

Selon l'invention, la composition peut contenir un ou plusieurs esters aromatiques, liquides à température ambiante (25 °C) et pression atmosphérique (760 mm de Hg).

Lorsque le groupe hydroxyle du composé aliphatique hydroxylé engagé dans l'estérification avec l'acide aromatique est en bout de chaîne, ce groupe est en position α-ω par rapport à la fonction -COOH de l'acide hydroxy-carboxylique aliphatique.

De préférence, les esters aromatiques selon l'invention présentent une viscosité supérieure à 500 cP (50 Pa.s) à 20°C mais aussi à 23°C, de préférence allant de 900 à 10 000 cP (90 à 1 000 Pa.s) et mieux de 950 à 5 000 cP (95 à 500 Pa.s), mesurée en particulier avec un viscosimètre type Brookfield RV ou un viscosimètre type Brookfield "DV-II+" de type LV équipé d'une aiguille n° 1 tournant entre 0,5 et 10 tr/min, au bout de 10 minutes et/ou un indice de réfraction ≥ 1,48 à 20°C; et notamment allant de 1,48 à 1,55 (l'indice de réfraction étant défini pour la raie D du sodium).

L'acide aromatique peut être choisi parmi les acides carboxyliques suivants :
a) les mono acides, tels que l'acide benzoïque, l'acide phényl acétique, l'acide cinnamique, l'acide 3 phényl propanoïque, l'acide salicylique ;
b) les diacides tels que l'acide téréphtalique ;
c) les triacides tels que l'acide trimellitique ; et
d) les tétra-acides tels que l'acide pyromellitique.

De façon avantageuse, l'acide carboxylique aromatique est l'acide benzoïque.

Comme ester utilisable dans l'invention, on peut citer ceux résultant de l'estérification par au moins un acide aromatique de l'un au moins des acides carboxyliques hydroxylés aliphatiques suivants :
i) les monoacides mono hydroxylés linéaires saturés de formule : 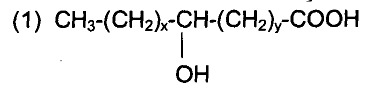 avec 0 ≤ x + y ≤ 37
   comme l'acide lactique (x + y = 0) ; l'acide 12-hydroxy octadécanoïque (ou 12-hydroxy stéarique) de formule : 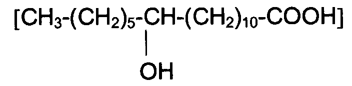 avec x = 5, y = 10 et x + y = 15
   et l'acide α-hydroxy octadécanoïque 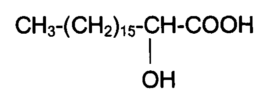 (avec x = 5 et y = 0)
   ou (2) HO-CH₂-(CH₂)ₓ-COOH avec 0 ≤ x ≤ 38
   comme l'acide glycolique HO-CH₂-COOH x = 0 ; ou l'acide junipérique (acide 16-hydroxy hexadécanoïque) de formule HO-CH₂-(CH₂)₁₄-COOH avec x = 14 ;
ii) les monoacides mono hydroxylés ramifiés saturés de formule : 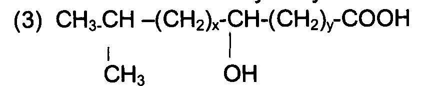 avec 0 ≤ x + y ≤ 35
   comme l'acide 5-méthyl 2-hydroxy hexanoïque (acide leucinique) de formule : 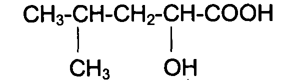 avec x = 1 y = 0 et x + y = 1
   ou encore l'acide 2-éthyl 3-hydroxy caprylique de formule : 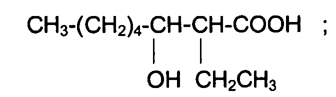
iii) Les monoacides mono hydroxylés insaturés de formule : 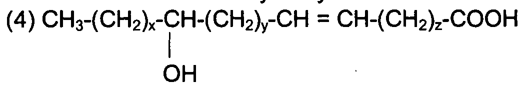 avec 0 ≤ x + y + z ≤ 35
   comme l'acide 12-hydroxy (cis) 9-octadécénoïque (ou l'acide ricinoléïque) de formule : 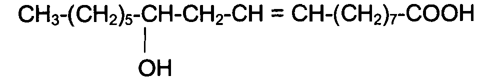 avec x = 5, y = 1, z = 7 et x + y + z = 13
   ou 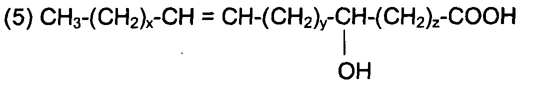 avec 0 ≤ x + y + z ≤ 35
   comme l'acide 3-hydroxy 4-hexanoïque de formule 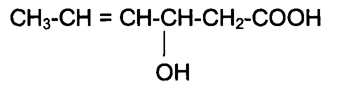 avec x = 0, y = 0, z = 1 et x + y + z = 1
   ou l'acide 2-hydroxy 15-tétracosènoïque (ou acide oxynervonique) de formule : 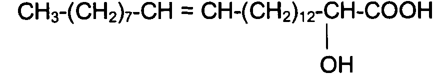 avec x = 7, y = 12, z = 0 et x + y + z = 17
   ou (6) HOCH₂-(CH₂)ₓ-CH = CH-(CH₂)_{y}-COOH avec 0 ≤ x + y ≤ 36
   comme l'acide 16-hydroxy 6-hexadécènoïque avec x = 8, y = 4 et x + y = 12 de formule HO-CH₂-(CH₂)₈-CH = CH-(CH₂)₄-COOH ;
iv) les monoacides poly hydroxylés saturés de formule : 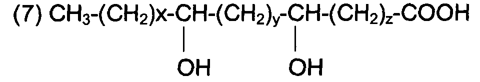 avec 0 ≤ x + y + z ≤ 36
   comme l'acide 9, 10-dihydroxy octadécanoïque de formule 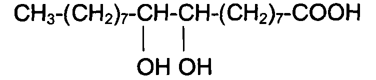 avec x = 7, y = 0, z = 7 et x + y + z = 14
   comme l'acide 9, 12-dihydroxy octadécanoïque de formule 7 avec x = 5, y = 2, z = 7 et x + y + z = 14
   ou l'acide 9, 10, 16-trihydroxy hexadécanoïque (acide aleuritique), l'acide 9, 10, 12-trihydroxy octadécanoïque de formule 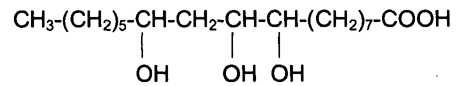 ou encore l'acide hexahydroxy octadécanoïque et l'acide octahydroxy octadécanoïque ;
v) les polyacides mono hydroxylés saturés de formule : 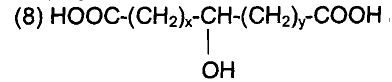 avec 0 ≤ x + y ≤ 37 comme l'acide malique,
   ou encore l'acide citrique ; et
vi) les polyacides poly hydroxylés saturés comme l'acide tartrique ;
   et leurs mélanges.
   Comme autre composé hydroxylé aliphatique utilisable dans l'invention, on peut citer ceux résultant de l'estérification par au moins un acide aromatique de l'un au moins des esters d'acides hydroxylés aliphatiques suivants :
vii) les esters de monoacides aliphatiques mono hydroxylés linéaires saturés tels que :
   - les esters de l'acide lactique comme le lactate d'isostéaryle, le lactate issu d'alcool en C₁₂-C₁₃, le lactate d'octyl dodécyle, le lactate d'oléyle, le lactate de myristyle ;
   - les esters de l'acide 12-hydroxy octadécanoïque (ou 12-hydroxy stéarique) comme l'hydroxy stéarate d'éthyl 2-hexyle, l'hydroxy stéarate d'octyl docécyle, l'hydroxy stéarate d'isostéaryle, l'hydroxy stéarate d'isodécyle, le trihydroxy stéarate de glycéryle (ou huile de ricin hydrogénée), l'hexahydroxy stéarate de dipentaérythrityle ;
viii) les esters de monoacides aliphatiques mono hydroxylés insaturés tels que les esters de l'acide ricinoléique (ou acide 12-hydroxy (cis) 9-octadécénoïque) comme le ricinoléate de butyle, le ricinoléate d'octyl dodécyle, le ricinoléate de cétyle, le triricinoléate de glycéryle (huile de ricin) ;
ix) les esters de polyacides aliphatiques mono hydroxylés saturés tels que le malate de diisostéaryle, le citrate de triisostéaryle, le citrate de trioctyl dodécyle ;
x) les esters de polyacides aliphatiques poly hydroxylés saturés comme le tartrate issu de la réaction avec 2 alcools en C₁₂-C₁₃ ramifiés ;
   et leurs mélanges.
   Comme composé hydroxylé sous forme d'ester, utilisable dans l'invention et résultant de l'estérification d'un polyol, on peut citer de façon générale :
xi) les esters partiels ou totaux de polyol en C₂ à C₁₆ ayant réagit avec un acide aliphatique hydroxylé comme notamment les triglycérides, les esters du pentaérythritol, du néopentyl glycol, du dipentaérythritol, du polyglycérol, les esters du sorbitol ;
et leurs mélanges.

De préférence, les esters aromatiques de l'invention sont choisis parmi les esters d'ester d'acides gras aliphatiques dont le reste d'acide gras comporte au moins 12 atomes de carbone. En particulier, le composé hydroxylé est choisi parmi les esters de l'acide ricinoléïque, les esters de l'acide 12-hydroxy stéarique, les esters de l'acide lactique, les esters de l'acide hydroxy 14-eicosènoïque et leurs mélanges.

Comme ester aromatique, on utilise notamment :
- l'ester résultant de la réaction d'estérification de l'huile de ricin avec l'acide benzoïque dans les proportions de 1 pour 1 (1/1) par exemple commercialisé par la société Finetex sous la référence Finsolv BCO-110 et qui sera appelé par la suite glycéryl monobenzoyl ricinoléate,
- le composé résultant de la réaction de l'huile de ricin avec l'acide benzoïque dans les proportions de 1 pour 1,5 (1/1,5) par exemple commercialisé par la société Finetex sous la référence Finsolv BCO-115 et qui sera aussi appelé par la suite glycéryl mono -dibenzoyl ricinoléate,
- le composé résultant de la réaction d'estérification de l'huile de ricin avec l'acide benzoïque dans les proportions de 1 pour 2 (1/2) par exemple commercialisé par la société Finetex sous la référence Finsolv BCO-120 et qui sera appelé par la suite glycéryl dibenzoyl ricinoléate,
- le composé résultant de la réaction d'estérification de l'huile de ricin avec l'acide benzoïque dans les proportions de 1 pour 3 (1/3) par exemple commercialisé par la société Finetex sous la référence Finsolv BCO-130 et qui sera appelé par la suite glycéryl tribenzoyl ricinoléate, et
- leurs mélanges.

L'acide ricinoléique représente de 80 à 92 % de l'huile de ricin. Ainsi son estérification conduit majoritairement (80 à 92 %) à l'ester de l'ester de l'acide ricinoléïque.

On peut aussi utiliser l'ester résultant de la réaction d'estérification avec de l'acide benzoïque de l'huile contenant majoritairement (52 à 57 %) un ester de l'acide hydroxy 14-eicosénoïque ou acide lesquerolique.

L'ester aromatique de la composition de l'invention peut être fabriqué selon le procédé décrit dans le document US-A-5 959 130.

L'ester aromatique de la composition de l'invention peut représenter de 0,1 à 99,9 % du poids total de la composition, de préférence de 1 à 99 %, mieux de 1 à 80 %, encore mieux de 10 à 40% et encore mieux de 15 à 25% et de façon générale, être présent en une quantité suffisante pour conférer à la composition des propriétés de non gras, de non collant, de glissant, de brillance, de couvrance, de stabilité et/ou de tenue dans le temps.

### Pâteux

Par "pâteux" au sens de la présente invention, on entend un composé gras lipophile à changement d'état solide/liquide réversible, présentant à l'état solide une organisation cristalline anisotrope, et comportant à la température de 23°C une fraction liquide et une fraction solide.

Par composé pâteux au sens de l'invention, on entend un composé ayant une dureté à 20°C allant de 0,001 à 0,5 MPa, de préférence de 0,002 à 0,4 MPa.

La dureté est mesurée selon une méthode de pénétration d'une sonde dans un échantillon de composé et en particulier à l'aide d'un analyseur de texture (par exemple le TA-XT2i de chez Rhéo) équipé d'un cylindre en inox de 2 mm de diamètre. La mesure de dureté est effectuée à 20°C au centre de 5 échantillons. Le cylindre est introduit dans chaque échantillon à une pré-vitesse de 1mm/s puis à une vitesse de mesure de 0,1 mm/s, la profondeur de pénétration étant de 0,3 mm. La valeur relevée de la dureté est celle du pic maximum.

Ce composé pâteux est en outre, à la température de 23°C, sous la forme d'une fraction liquide et d'une fraction solide. En d'autres termes, la température de fusion commençante du composé pâteux est inférieure à 23°C. La fraction liquide du composé pâteux mesurée à 23°C représente 9 à 97% en poids du composé. Cette fraction liquide à 23°C représente de préférence entre 15 et 85%, de préférence encore entre 40 et 85% en poids.

La fraction liquide en poids du composé pâteux à 23°C est égale au rapport de l'enthalpie de fusion consommée à 23°C sur l'enthalpie de fusion du composé pâteux.

L'enthalpie de fusion du composé pâteux est l'enthalpie consommée par le composé pour passer de l'état solide à l'état liquide. Le composé pâteux est dit à l'état solide lorsque l'intégralité de sa masse est sous forme solide cristalline. Le composé pâteux est dit à l'état liquide lorsque l'intégralité de sa masse est sous forme liquide.

L'enthalpie de fusion du composé pâteux est égale à l'aire sous la courbe du thermogramme obtenu à l'aide d'un calorimètre à balayage différentiel (D. S. C), tel que le calorimètre vendu sous la dénomination MDSC 2920 par la société TA instrument, avec une montée en température de 5 ou 10°C par minute, selon la norme ISO 11357-3:1999. L'enthalpie de fusion du composé pâteux est la quantité d'énergie nécessaire pour faire passer le composé de l'état solide à l'état liquide. Elle est exprimée eu J/g.

L'enthalpie de fusion consommée à 23°C est la quantité d'énergie absorbée par l'échantillon pour passer de l'état solide à l'état qu'il présente à 23°C constitué d'une fraction liquide et d'une fraction solide.

La fraction liquide du composé pâteux mesurée à 32°C représente de préférence de 30 à 100% en poids du composé, de préférence de 80 à 100%, de préférence encore de 90 à 100% en poids du composé. Lorsque la fraction liquide du composé pâteux mesurée à 32°C est égale à 100%, la température de la fin de la plage de fusion du composé pâteux est inférieure ou égale à 32°C.

La fraction liquide du composé pâteux mesurée à 32°C est égale au rapport de l'enthalpie de fusion consommée à 32°C sur l'enthalpie de fusion du composé pâteux. L'enthalpie de fusion consommée à 32°C est calculée de la même façon que l'enthalpie de fusion consommée à 23°C.

Le composé pâteux est de préférence choisi parmi les composés synthétiques et les composés d'origine végétale. Un composé pâteux peut être obtenu par synthèse à partir de produits de départ d'origine végétale.

Le composé pâteux est avantageusement choisi parmi
- les composés siliconés polymères ou non
- les composés fluorés polymères ou non
- les polymères vinyliques, notamment:
   - les homopolymères d'oléfines
   - les copolymères d'oléfines
   - les homopolymères et copolymères de diènes hydrogénés
   - les oligomères linéaires ou ramifiés, homo ou copolymères de (méth)acrylates d'alkyles ayant de préférence un groupement alkyle en C₈-C₃₀
   - les oligomères homo et copolymères d'esters vinyliques ayant des groupements alkyles en C₈-C₃₀
   - les oligomères homo et copolymères de vinyléthers ayant des groupements alkyles en C₈-C₃₀,
- les polyéthers liposolubles résultant de la polyéthérification entre un ou plusieurs diols en C2-C100, de préférence en C2-C50,
- les esters,
et leurs mélanges.

Le composé pâteux est de préférence polymère, notamment hydrocarboné.

### Composés pâteux siliconés et /ou fluorés

Un composé pâteux siliconé et fluoré préféré est le polyméthyl trifluoropropryl méthylalkyl diméthylsiloxane, fabriqué sous la dénomination X22-1088 par SHIN ETSU.

Lorsque le composé pâteux est un polymère siliconé et/ou fluoré, la composition comprend avantageusement un agent compatibilisant tel que les esters à courte chaîne comme le néopentanoate d'isodécyle.

### Composés pâteux polyéthers

Parmi les polyéthers liposolubles, on préfère en particulier les copolymères d'éthylène-oxyde et/ou de propylène-oxyde avec des alkylènes-oxydes à longue chaîne en C6-C30, de préférence encore tels que le rapport pondéral de l'éthylène-oxyde et/ou de propylène-oxyde avec alkylènes-oxydes dans le copolymère est de 5:95 à 70:30. Dans cette famille, on citera notamment les copolymères tels que les alkylènes-oxydes à longue chaîne sont disposés en blocs ayant un poids moléculaire moyen de 1.000 à 10.000, par exemple un copolymère bloc de polyoxyethylène/polydodécyle glycol tel que les éthers de dodécanediol (22 mol) et de polyéthylène glycol (45 OE) commercialisés sous la marque ELFACOS ST9 par Akzo Nobel.

Parmi les esters, on préfère notamment
- les esters d'acides gras végétaux, iso-stéarique, adipique de glycéryle commercialisés sous la marque Softisan 649 par Sasol,
- le propionate d'arachidyle commercialisé sous la marque Waxenol 801 par Alzo,
- les esters de phytostérol,
- les polyesters non réticulés résultant de la polycondensation entre un diacide ou un polyacide carboxylique linéaire ou ramifié en C4-C50 et un diol ou un polyol en C2-C50,
- les esters aliphatiques d'ester résultant de l'estérification d'un ester d'acide hydroxycarboxylique aliphatique par un acide carboxylique aliphatique;
L'acide carboxylique aliphatique comprend de 4 à 30 et de préférence de 6 à 30 atomes de carbone. Il est de préférence choisi parmi l'acide héxanoïque, l'acide heptanoïque, l'acide octanoïque, l'acide éthyl-2 héxanoïque, l'acide nonanoïque, l'acide décanoïque, l'acide undécanoïque, acide dodécanoïque, l'acide tridécanoïque, l'acide tétradécanoïque, l'acide pentadécanoïque, l'acide héxadécanoïque, l'acide héxyldécanoïque, l'acide heptadécanoïque, l'acide octadécanoïque, l'acide isostéarique, l'acide nonadécanoïque, l'acide eicosanoïque, l'acide isoarachidique, l'acide octyldodécanoïque, l'acide henéicosanoïque, l'acide docosanoïque, et leurs mélanges.
L'acide carboxylique aliphatique est de préférence ramifié.
L'ester d'acide hydroxy carboxylique aliphatique est avantageusement issu d'un acide carboxylique aliphatique hydroxylé comportant de 2 à 40 atomes de carbone, de préférence de 10 à 34 atomes de carbone et mieux de 12 à 28 atomes de carbone, et de 1 à 20 groupes hydroxyle, de préférence de 1 à 10 groupes hydroxyle et mieux de 1 à 6 groupes hydroxyle. L'ester d'acide hydroxy carboxylique aliphatique est choisi parmi :
a) les esters partiels ou totaux de monoacides carboxyliques aliphatiques mono hydroxylés linéaires, saturés ;
b) les esters partiels ou totaux de monoacides carboxyliques aliphatiques mono hydroxylés insaturés ;
c) les esters partiels ou totaux de polyacides carboxyliques aliphatiques mono hydroxylés saturés ;
d) les esters partiels ou totaux de polyacides carboxyliques aliphatiques poly hydroxylés saturés ;
e) les esters partiels ou totaux de polyols aliphatiques en C₂ à C₁₆ ayant réagi avec un mono ou un polyacide carboxylique aliphatique mono ou poly hydroxylé,
et leurs mélanges.

Les esters aliphatiques d'ester sont avantageusement choisis parmi :
- l'ester résultant de la réaction d'estérification de l'huile de ricin hydrogénée avec l'acide isostéarique dans les proportions 1 pour 1 (1/1) ou monoisostéarate d'huile de ricin hydrogénée,
- l'ester résultant de la réaction d'estérification de l'huile de ricin hydrogénée avec l'acide isostéarique dans les proportions 1 pour 2 (1/2) ou le diisostéarate d'huile de ricin hydrogénée,
- l'ester résultant de la réaction d'estérification de l'huile de ricin hydrogénée avec l'acide isostéarique dans les proportions 1 pour 3 (1/3) ou triisostéarate d'huile de ricin hydrogénée,
- et leurs mélanges.

Parmi les composés pâteux d'origine végétale, on choisira de préférence un mélange de stérols de soja et de pentaérythritol oxyéthyléné (5OE) oxypropyléné (5 OP), commercialisé sous la référence Lanolide par la société VEVY.

Le composé pâteux représente de préférence 1 à 99%, mieux 1 à 60%, mieux 2 à 30% et mieux encore 5 à 15% en poids de la composition.

### Formes de la composition

La composition de l'invention peut se présenter sous la forme de composition solide, compactée ou coulée en stick ou en coupelle, pâteuse ou liquide. Avantageusement, elle se présente sous forme solide, à savoir sous forme dure (ne s'écoulant pas sous son propre poids) notamment coulée ou compactée, par exemple en stick ou en coupelle.

Elle peut se présenter sous forme de pâte, de solide ou de crème. Elle peut être une émulsion huile-dans-eau ou eau-dans-huile, un gel anhydre, solide ou souple ou encore sous forme de poudre libre ou compactée et même sous forme biphasique. De préférence, elle se présente sous forme de composition à phase continue huileuse et notamment anhydre ; dans ce cas, elle peut contenir une phase aqueuse à un taux inférieur à 10 % et mieux inférieur à 5% en poids.

### Colorants

Avantageusement, la composition de l'invention peut comprendre, en outre, au moins une matière colorante qui peut être choisie parmi les colorants solubles ou dispersibles dans la composition, les pigments, les nacres et leurs mélanges. Les colorants sont de préférence des colorants liposolubles, bien que les colorants hydrosolubles puissent être utilisés. Cette matière colorante peut représenter de 0,001 à 98 %, de préférence de 0,5 à 85% et mieux de 1 à 60 % du poids total de la composition.
Pour une composition sous forme de pâte ou coulée telle que les rouges à lèvres ou les produits de maquillage du corps, on utilise en général de 0,5 à 50% de matière colorante, de préférence de 2 à 40 % et mieux de 5 à 30%, par rapport au poids total de la composition.

Les colorants liposolubles sont par exemple le rouge Soudan, le D & C Red 17, le D & C Green 6, le β-carotène, l'huile de soja, le brun Soudan, le D & C Yellow 11, le D & C Violet 2, le D & C orange 5, le jaune quinoléine, le rocou. Ils peuvent représenter de 0 à 20 % du poids de la composition et mieux de 0,1 à 6 %. Les colorants hydrosolubles sont notamment le jus de betterave, le bleu de méthylène et peuvent représenter de 0,1 à 6 % en poids de la composition (si présents).

De préférence, la composition de l'invention, comprend une phase particulaire avantageusement colorée pouvant représenter de 0,001 à 50 % du poids total de la composition, de préférence de 0,01 à 40 % et mieux de 0,05 à 30 %, et qui peut comprendre des pigments et/ou des nacres et/ou des charges habituellement utilisés dans les compositions cosmétiques.

Par pigments, il faut comprendre des particules blanches ou colorées, minérales ou organiques, insolubles dans la phase grasse liquide, destinées à colorer et/ou opacifier la composition. Par charges, il faut comprendre des particules incolores ou blanches, minérales ou de synthèse, lamellaires ou non lamellaires. Par nacres, il faut comprendre des particules irisées, notamment produites par certains mollusques dans leur coquille ou bien synthétisées. Ces charges et nacres servent notamment à modifier la texture de la composition.

Les pigments peuvent être présents dans la composition à raison de 0,05 à 30 % (si présents) du poids de la composition finale, et de préférence à raison de 2 à 20 %. Comme pigments minéraux utilisables dans l'invention, on peut citer les oxydes de titane, de zirconium ou de cérium ainsi que les oxydes de zinc, de fer ou de chrome et le bleu ferrique. Parmi les pigments organiques utilisables dans l'invention, on peut citer le noir de carbone, et les laques de baryum, strontium, calcium (D & C Red N°7), aluminium.

Les nacres peuvent être présentes dans la composition à raison de 0,001 à 20 % (si présentes) du poids total de la composition, de préférence à un taux de l'ordre de 1 à 15 %. Parmi les nacres utilisables dans l'invention, on peut citer le mica recouvert d'oxyde de titane, d'oxyde de fer, de pigment naturel ou d'oxychlorure de bismuth tel que le mica titane coloré, les pigments goniochromatiques et par exemple les pigments multicouches interférentiels.

Les charges peuvent être présentes à raison de 0,001 à 35 % (si présentes) du poids total de la composition, de préférence 0,5 à 15 %. On peut notamment citer le talc, le mica, le kaolin, les poudres de Nylon® (Orgasol notamment) et de polyéthylène, les poudres de polytétrafluoroéthylène (Téflon®), l'amidon, le nitrure de bore, des microsphères de copolymères telles que l'Expancel® (Nobel Industrie), le Polytrap® (Dow Corning), le Polypore® L 200 (Chemdal Corporation) et les microbilles de résine de silicone (Tospearl ® de Toshiba, par exemple), la sillice.

La composition selon l'invention peut contenir au moins un composé non aqueux additionnel choisi parmi les huiles, les corps gras pâteux à température ambiante, les cires, les gommes, les résines, les polymères lipophiles et leurs mélanges.

### Cires et gommes

En particulier, la composition peut contenir au moins une cire.

Par "cire" au sens de la présente invention, on entend un composé gras lipophile, solide à température ambiante (25°C), à changement d'état solide/liquide réversible, ayant une température de fusion supérieure à 30°C pouvant aller jusqu'à 200° C, une dureté supérieure à 0,5 MPa, et présentant à l'état solide une organisation cristalline anisotrope. En portant la cire à sa température de fusion, il est possible de la rendre miscible aux huiles et de former un mélange homogène microscopiquement, mais en ramenant la température du mélange à la température ambiante, on obtient une recristallisation de la cire dans les huiles du mélange.

Les cires utilisables dans l'invention sont des composés solides à température ambiante, destinés à structurer la composition en particulier sous forme de stick ; elles peuvent être hydrocarbonées, fluorées et/ou siliconées et être d'origine végétale, minérale, animale et/ou synthétique. En particulier, elles présentent une température de fusion supérieure à 40°C et mieux supérieure à 45°C.

Comme cire utilisable dans l'invention, on peut citer celles généralement utilisées dans le domaine cosmétique : elles sont notamment d'origine naturelle comme la cire d'abeilles, la cire de Carnauba, de Candelilla, d'Ouricoury, du Japon, de fibres de liège ou de canne à sucre, de riz, de Montan, la paraffine, les cires de lignite ou microcristalline, la cérésine ou l'ozokérite, les huiles hydrogénées comme l'huile de jojoba ; les cires synthétiques comme les cires de polyéthylène issues de la polymérisation ou copolymérisation de l'éthylène et les cires de Fischer-Tropsch ou encore des esters d'acides gras comme l'octacosanyl stéarate, les glycérides concrets à 40°C et mieux à 45°C, les cires de silicones comme les alkyl- ou alkoxydiméthicones ayant une chaîne alkyle ou alcoxy de 10 à 45 atomes de carbone, les esters de poly(di)méthylsiloxane solide à 40°C dont la chaîne ester comporte au moins 10 atomes de carbone ; et leurs mélanges.

Les gommes utilisables dans l'invention se présentent généralement sous forme solubilisée dans une huile, les polymères sont solides à température ambiante et les résines peuvent être liquides ou solides à température ambiante.

Par gomme on entend un corps gras qui se présente sous forme de polymère solide à température ambiante ayant un poids moléculaire moyen en poids de 50 000 à 1 000 000. La gomme est souvent vendue en dispersion dans un solvant organique, du type huile de silicone.

La nature et la quantité des gommes ou cires sont fonction des propriétés mécaniques et des textures recherchées. A titre indicatif, la cire peut représenter de 0,01 à 50 %, de préférence de 2 à 40 %, et mieux de 5 à 30 % du poids total de la composition.

La dureté peut être mesurée par la méthode dite "du fil à couper le beurre", qui consiste à couper un bâton de rouge à lèvres de 12,7 mm et à mesurer la dureté à 20°C, au moyen d'un dynamomètre DFGHS 2 de la société Indelco-Chatillon se déplaçant à une vitesse de 100 mm/minute. Elle est exprimée comme la force de cisaillement (exprimée en gramme) nécessaire pour couper un stick dans ces conditions. Selon cette méthode la dureté d'une composition en stick selon l'invention va notamment de 50 à 300 g, de préférence de 100 à 250 g et par exemple de 150 à 230 g.

### Huiles

Par « huile » on entend un corps gras liquide à température ambiante et pression atmosphérique.

Les huiles peuvent être des huiles hydrocarbonées et/ou siliconées et/ou fluorées. Ces huiles peuvent être d'origine animale, végétale, minérale ou synthétique.

"Par huile hydrocarbonée", on entend une huile comportant principalement des atomes de carbone et d'hydrogène et éventuellement une ou plusieurs fonctions choisies parmi les fonctions hydroxyle, ester, éther, carboxylique. A titre d'exemple d'huile utilisable dans l'invention, on peut citer :
- les huiles hydrocarbonées d'origine animale telles que le perhydrosqualène ;
- les huiles hydrocarbonées végétales telles que les triglycérides liquides d'acides gras de 4 à 24 atomes de carbone comme les triglycérides des acides heptanoïque ou octanoïque ou encore les huiles de tournesol, de maïs, de soja, de courge, de pépins de raisin, de sésame, de noisette, d'abricot, de macadamia, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, de beurre de karité ;
- les hydrocarbures linéaires ou ramifiés, d'origine minérale ou synthétique tels que les huiles de paraffine et leurs dérivés, la vaseline, les polydécènes, le polyisobutène hydrogéné tel que le Parleam ;
- les esters et les éthers de synthèse notamment d'acides gras comme les huiles de formule R₁COOR₂ dans laquelle R₁ représente le reste d'un acide gras supérieur comportant de 1 à 40 atomes de carbone et R₂ représente une chaîne hydrocarbonée contenant de 1 à 40 atomes de carbone avec R₁ + R₂ ≥ 10 comme par exemple l'huile de Purcellin, l'isononanoate d'isononyle, le myristate d'isopropyle, le palmitate d'éthyl 2-hexyle, le stéarate d'octyl 2-dodécyle, l'érucate d'octyl 2-dodécyle, l'isostéarate d'isostéaryle ; les esters hydroxylés comme l'isostéaryl lactate, l'octyl hydroxy stéarate, l'hydroxy stéarate d'octyl dodécyle, le diisostéaryl malate, le citrate de triisocétyle, des heptanoates, octanoates, décanoates d'alcools gras ; des esters de polyol comme le dioctanoate de propylène glycol, le diheptanoate de néopentyl glycol, le diisononanoate de diéthylène glycol ; et les esters du pentaérythritol comme le tétra-isostéarate de pentaérythrytyle ;
- des alcools gras ayant de 12 à 26 atomes de carbone comme l'octyl dodécanol, le 2-butyloctanol, le 2-hexyl décanol, le 2-undécyl pentadécanol, l'alcool oléique ;
- les huiles fluorées éventuellement partiellement hydrocarbonées et/ou siliconées ;
- les huiles siliconées comme les polydiméthylsiloxanes (PDMS) volatiles ou non, linéaires ou cycliques ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényl triméthicones, les phényl diméthicones, les phényl triméthylsiloxy diphényl siloxanes, les diphényl diméthicones, les diphényl méthyldiphényl trisiloxanes, les 2-phényl éthyl triméthyl-siloxysilicates,
- leurs mélanges.

Les huiles additionnelles peuvent représenter de 0 à 90 % du poids total de la composition, de préférence de 0,05 à 60 % et mieux de 1 à 35 %.

On préfère dans le cadre de la présente invention des huiles additionnelles dont le poids moléculaire est compris entre 650 et 10000 g/mol, de préférence entre 750 et 7.500 g/mol.

Selon un mode de mise en oeuvre, la composition de l'invention comprend une phase huileuse comprenant au moins 70% en poids d'une huile de masse molaire comprise entre 650 et 10.000 g/mol, de préférence entre 750 et 7.500 g/mol. La phase huileuse comprend avantageusement plus de 80%, de préférence plus de 85% en poids d'une huile de masse molaire comprise entre 650 et 10.000 g/mol, de préférence entre 750 et 7.500 g/mol.

L'huile de masse molaire élevée est de préférence choisie parmi les polymères lipophiles
- les esters d'acides gras linéaires ayant un nombre total de carbones allant de 35 à 70
- les esters hydroxylés
- les esters aromatiques
- les esters d'alcool gras ou d'acides gras ramifiés en C24-C28
- les huiles siliconées
- les huiles d'origine végétale
et leurs mélanges.

L'huile de masse molaire élevée est de préférence choisie parmi les polybutylènes, les polyisobutylènes hydrogénés, les polydécènes, les polydécènes hydrogénés, les copolymères de la vinylpyrrolidone tel que le PVP / héxadécène copolymère, le tétrapélargonate de pentaérythrityle, le triisostéarate de polyglycérol-2, le tridécyl trimellitate, le citrate de triisoarachidyle, le tétraisononanoate de pentaérythrityle, le triisostéarate de pentaérythrityle, le tétraisostéarate de polyglycéryle-2, le tétra décyl-2 tétradécanoate de pentaérythrityle, les silicones phénylées, l'huile de sésame, et leurs mélanges.

### Additifs

La composition de l'invention peut comprendre, en outre, tout additif usuellement utilisé dans le domaine concerné, tel que l'eau, des antioxydants, des conservateurs, des neutralisants, des gélifiants lipophiles ou de corps gras liquides, des gélifiants de phase aqueuse, des dispersants, des actifs cosmétiques ou dermatologiques. Ces additifs, à l'exception de l'eau qui peut représenter de 0 à 70 % et par exemple de 1 à 50 %,et mieux ce 1 à 10 % du poids total de la composition, peuvent être présents dans la composition à raison de 0 à 20% du poids total de la composition et mieux de 0 à 10%.

La composition selon l'invention peut se présenter sous la forme d'une composition, colorée ou non, sous forme d'une composition de protection solaire ou de démaquillage ou encore sous forme d'une composition hygiénique. Elle contient notamment des actifs cosmétiques. Elle peut alors être utilisée comme base de soin ou de traitement pour la peau comme les mains ou le visage ou pour les lèvres (baumes à lèvres, protégeant les lèvres du froid et/ou du soleil et/ou du vent) ou encore comme déodorant. Comme actif cosmétique utilisable dans l'invention, on peut citer les vitamines A, E, C, B₃, les provitamines comme le D-panthénol, les actifs apaisants comme l'α-bisabolol, l'aloe vera, l'allantoïne, les extraits de plantes ou les huiles essentielles, les agents protecteurs ou restructurants comme les céramides, les actifs fraîcheur comme le menthol et ses dérivés, les émollients (beurre de cacao, diméthicone), les hydratants (arginine PCA), les actifs antirides, les acides gras essentiels, et leurs mélanges.

La composition de l'invention peut également se présenter sous la forme d'un produit de maquillage de la peau, en particulier du visage comme un fond de teint, un blush, un fard comme un produit de tatouage semi-permanent ou de maquillage des lèvres comme un rouge ou un brillant à lèvres, présentant éventuellement des propriétés de soin ou de traitement, un produit de maquillage des phanères comme par exemple un vernis à ongles, un mascara, un eyeliner, un produit de coloration ou de soin des cheveux.

Bien entendu la composition de l'invention doit être cosmétiquement acceptable, à savoir non toxique et susceptible d'être appliquée sur la peau, les phanères ou les lèvres d'êtres humains.

La composition selon l'invention peut être fabriquée par les procédés connus, généralement utilisés dans le domaine cosmétique ou dermatologique.

L'invention a encore pour objet l'utilisation (i) d'au moins un ester à groupement aromatique, liquide à température ambiante, résultant de l'estérification par un acide aromatique d'au moins un groupe hydroxyle pendant ou en bout de chaîne d'un composé aliphatique hydroxylé choisi parmi les acides aliphatiques hydroxylés et leurs esters, et (ii) d'au moins un composé pâteux différent de la lanoline ou de l'un de ses dérivés ayant une dureté à 25°C comprise entre 0,001 à 0,5 MPa, de préférence de 0,002 à 0,4 MPa, et dont la fraction liquide à 23°C est comprise entre 9 et 97% en poids, de préférence entre 15 et 85%, de préférence encore entre 40 et 85% en poids,
dans une composition
pour conférer à un film de ladite composition des propriétés de non collant, de non gras, de brillance, de confort, de tenue dans le temps, de bon étalement et/ou de glissant, et pour limiter l'exsudation de ladite composition.

L'invention est illustrée plus en détail dans les exemples suivants. Les quantités sont données en pourcentage massique.

### Exemple 1 : Rouge à lèvres

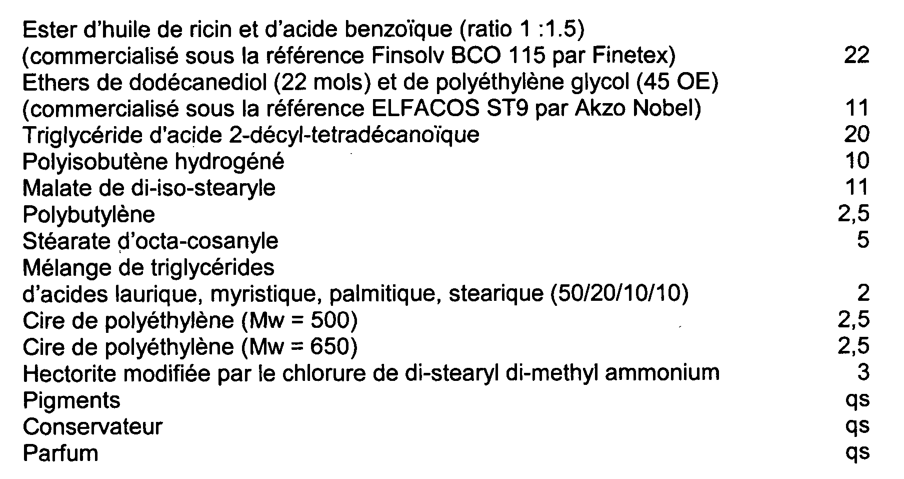

- La phase huileuse est réalisée en mélangeant le conservateur, toutes les huiles (ester d'huile de ricin et d'acide benzoïque), triglycéride d'acide 2-decyl tetradécanoïque, polyisobutène hydrogéné, malate de di-iso-stéaryle, polybutylène) ainsi que le pâteux (éthers de dodécanediol (22 mols) et de polyéthylène glycol(45 OE)).
- Puis l'hectorite est broyée dans la phase huileuse à la tricylindre (1 passage).
- Les pigments sont ensuite broyés dans le mélange hectorite + phase huileuse (3 passages).
- On ajoute le mélange obtenu dans un poélon avec les cires et on chauffe à 105°C pendant deux heures en homogénéïsant à l'aide d'un appareil Raynerie.
- On ajoute enfin le parfum, on homogénéïse 5 minutes puis on coule le mélange dans un moule à 42°C qui est placé à -20°C pendant 30 minutes. Puis on procède au démoulage des sticks.

La formule ci-dessus possède de bonnes propriétés en terme d'application (glissant), de confort, de brillance et de tenue dans le temps.

### Exemple 2 comparatif :

La formule ci-dessus a été reproduite en remplaçant le composé pâteux constitué de 11 % d'éther de dodécanediol (22 mols) et de polyéthylène glycol (45 OE) par 11 % de lanoline. La composition exempte de lanoline selon l'invention est plus glissante à l'application et moins collante, tout en étant aussi brillante. En outre le film tient mieux une heure après l'application.

## Revendications

1. Composition cosmétique de soin ou de maquillage des matières kératiniques, contenant au moins un ester à groupement aromatique, liquide à température ambiante, résultant de l'estérification par un acide aromatique d'au moins un groupe hydroxyle pendant ou en bout de chaîne d'un composé aliphatique hydroxylé choisi parmi les acides aliphatiques hydroxylés et leurs esters, ladite composition étant exempte de lanoline ou de dérivés de lanoline.

2. Composition selon la revendication 1, contenant au moins un composé pâteux différent de la lanoline ou de l'un de ses dérivés.

3. Composition selon l'une des revendications précédentes, **caractérisée en ce que** l'acide aromatique de l'ester à groupement aromatique est choisi parmi l'acide benzoïque, l'acide phényl acétique, l'acide cinnamique, l'acide 3 phényl propanoïque, l'acide salicylique ; l'acide téréphtalique ; l'acide trimellitique ; l'acide pyromellitique.

4. Composition selon l'une des revendications précédentes, **caractérisée en ce que** l'acide aromatique est l'acide benzoïque.

5. Composition selon l'une des revendications précédentes, **caractérisée en ce que** les acides aliphatiques hydroxylés comprennent de 2 à 40 atomes de carbone, de préférence de 10 à 34 atomes de carbone et mieux de 12 à 28 atomes de carbone.

6. Composition selon l'une des revendications précédentes, **caractérisée en ce que** les acides aliphatiques hydroxylés comportent, en outre, de 1 à 20 groupes hydroxyle, de préférence de 1 à 10 groupes hydroxyle et mieux de 1 à 6 groupes hydroxyle, susceptible(s) d'être estérifié(s) par l'acide aromatique.

7. Composition selon l'une quelconque des revendications précédentes, **caractérisée en ce que** le composé aliphatique hydroxylé est choisi parmi :
i) les monoacides aliphatiques mono hydroxylés linéaires saturés de formule : 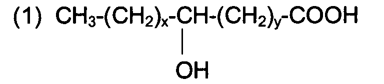 avec 0 ≤ x + y ≤ 37
ou (2) HO-CH₂-(CH₂)ₓ-COOH avec 0 ≤ x ≤ 38 ;
ii) les monoacides aliphatiques mono hydroxylés ramifiés saturés de formule : 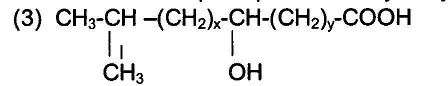 avec 0 ≤ x + y ≤ 35
Ou (3') l'acide 2-éthyl 3-hydroxy caprylique de formule : 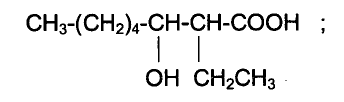
iii) les monoacides aliphatiques mono hydroxylés insaturés de formule : 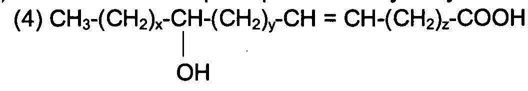 avec 0 ≤ x + y + z ≤ 35
ou 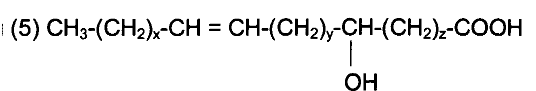 avec 0 ≤ x + y + z ≤ 35
ou (6) HOCH₂-(CH₂)ₓ-CH = CH-(CH₂)_{y}-COOH avec 0 ≤ x + y ≤ 36 ;
iv) les monoacides aliphatiques poly hydroxylés saturés de formule : 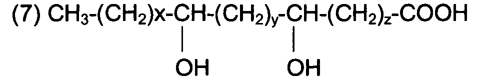 avec 0 ≤ x + y + z ≤ 36 ;
v) les polyacides aliphatiques mono hydroxylés saturés de formule : 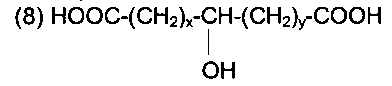 avec 0 ≤ x + y ≤ 37 ;
vi) les polyacides aliphatiques poly hydroxylés saturés ;
vii) les esters de monoacides aliphatiques mono hydroxylés linéaires saturés comme les esters de l'acide lactique et les esters de l'acide 12-hydroxy octadécanoïque ;
viii) les esters de monoacides aliphatiques mono hydroxylés insaturés tels que les esters de l'acide ricinoléique ;
ix) les esters de polyacides aliphatiques mono hydroxylés saturés ;
x) les esters de polyacides aliphatiques poly hydroxylés saturés ;
xi) les esters partiels ou totaux de polyol en C₂ à C₁₆ ayant réagit avec un acide aliphatique hydroxylé ;
et leurs mélanges.

8. Composition selon l'une quelconque des revendications précédentes, **caractérisée en ce que** le composé hydroxylé aliphatique est choisi parmi :
- l'acide lactique ; l'acide 12-hydroxy octadécanoïque ; l'acide α-hydroxy octadécanoïque ;
- l'acide glycolique ou l'acide junipérique ;
- l'acide leucinique ou l'acide 2-éthyl 3-hydroxy caprylique ;
- l'acide ricinoléïque ;
- l'acide 3-hydroxy 4-hexanoïque ou l'acide oxynervonique ;
- l'acide 16-hydroxy 6-hexadécènoïque ;
- l'acide 9, 10-dihydroxy octadécanoïque, l'acide 9, 12-dihydroxy octadécanoïque, l'acide aleuritique, l'acide 9, 10, 12-trihydroxy octadécanoïque, l'acide hexahydroxy octadécanoïque ou l'acide octahydroxy octadécanoïque ;
- l'acide malique ou l'acide citrique ;
- l'acide tartrique ;
- le lactate d'isostéaryle, le lactate issu d'alcool en C₁₂-C₁₃, le lactate d'octyl dodécyle, le lactate d'oléyle, le lactate de myristyle ;
- l'hydroxy stéarate d'éthyl 2-hexyle, l'hydroxy stéarate d'octyl docécyle, l'hydroxy stéarate d'isostéaryle, l'hydroxy stéarate d'isodécyle, le trihydroxy stéarate de glycéryle, l'hexahydroxy stéarate de dipentaérythrityle ;
- le ricinoléate de butyle, le ricinoléate d'octyl dodécyle, le ricinoléate de cétyle, le triricinoléate de glycéryle ;
- le malate de diisostéaryle, le citrate de triisostéaryle, le citrate de trioctyl dodécyle ;
- le tartrate issu de dialcools en C₁₂-C₁₃ ramifiés ;
- et leurs mélanges.

9. Composition selon l'une des revendications précédentes, **caractérisée en ce que** l'ester aromatique est un ester d'ester d'acide gras aliphatique dont le reste d'acide gras comporte au moins 12 atomes de carbone.

10. Composition selon l'une quelconque des revendications précédentes, **caractérisée en ce que** le composé hydroxylé aliphatique est choisi parmi les esters de l'acide ricinoléïque, les esters de l'acide 12-hydroxy stéarique, les esters de l'acide lactique, les esters de l'acide hydroxy 14-eicosènoïque et leurs mélanges.

11. Composition selon l'une des revendications précédentes, **caractérisée en ce que** l'ester aromatique présente une viscosité supérieure à 500 cP (50 Pa.s) à 20°C, de préférence allant de 900 à 10 000 cP (90 à 1 000 Pa.s) et mieux de 950 à 5 000 cP (95 à 500 Pa.s) et/ou un indice de réfraction ≥ 1,48.

12. Composition selon l'une des revendications précédentes, **caractérisée en ce que** l'ester aromatique est choisi parmi le glycéryl monobenzoyl ricinoléate, le glycéryl monodibenzoyl ricinoléate, le glycéryl dibenzoyl ricinoléate, glycéryl tribenzoyl ricinoléate et leurs mélanges.

13. Composition selon l'une des revendications précédentes, **caractérisée en ce que** l'ester aromatique est présent en une quantité suffisante pour conférer à la composition des propriétés de non gras, de non collant, de glissant, de brillance, de couvrance, de non exsudation et/ou de tenue dans le temps.

14. Composition selon l'une des revendications précédentes, **caractérisée en ce que** l'ester aromatique est présent en une quantité allant de 0,1 à 99,9 %, de préférence de 1 à 99 % et mieux de 5 à 90% du poids total de la composition.

15. Composition selon la revendication 2, **caractérisée en ce que** le composé pâteux a une dureté à 20°C comprise entre 0,001 à 0,5 MPa, de préférence de 0,002 à 0,4 MPa.

16. Composition selon la revendication 2, **caractérisée en ce que** le composé pâteux a une fraction liquide à 23°C comprise entre 9 et 97% en poids, de préférence entre 15 et 85%, de préférence encore entre 40 et 85% en poids.

17. Composition selon la revendication 2, **caractérisée en ce que** le composé pâteux a une fraction liquide à 32°C comprise entre 30 et 100% en poids, de préférence entre 80 et 100%, de préférence encore entre 90 et 100% en poids.

18. Composition selon la revendication 2, **caractérisée en ce que** le composé pâteux est choisi parmi
- les composés siliconés polymères ou non,
- les composés fluorés polymères ou non,
- les polymères vinyliques, notamment:
• les homopolymères d'oléfines
• les copolymères d'oléfines
• les homopolymères et copolymères de diènes hydrogénés
• les oligomères linéaires ou ramifiés, homo ou copolymères de (méth)acrylates d'alkyles ayant de préférence un groupement alkyle en C₈-C₃₀
• les oligomères homo et copolymères d'esters vinyliques ayant des groupements alkyles en C₈-C₃₀
• les oligomères homo et copolymères de vinyléthers ayant des groupements alkyles en C₈-C₃₀,
- les polyéthers liposolubles résultant de la polyéthérification entre un ou plusieurs diols en C2-C100, de préférence en C2-C50,
- les esters,
et leurs mélanges.

19. Composition selon la revendication 2 **caractérisée en ce que** le composé pâteux est hydrocarboné.

20. Composition selon la revendication 18, **caractérisée en ce que** le composé pâteux est le polyméthyl trifluoropropryl méthylalkyl diméthylsiloxane.

21. Composition selon la revendication 18, **caractérisée en ce que** le polyéther liposoluble est choisi parmi les copolymères d'éthylène-oxyde et/ou de prolylène-oxyde avec des alkylènes oxydes à longue chaîne en C6-C30, de préférence encore tels que le rapport pondéral de l'éthylène-oxyde et/ou de propylène-oxyde aux alkylènes-oxydes dans le copolymère est de 5:95 à 70:30.

22. Composition selon la revendication 21, **caractérisée en ce que** le polyéther liposoluble est un copolymère bloc de polyoxyethylène/polydodécyle glycol.

23. Composition selon la revendication 18, **caractérisée en ce que** les esters sont choisis parmi
- les esters d'acides gras végétaux, notamment l'iso-stéarate ou l'adipate de glycéryle,
- le propionate d'arachidyle,
- les esters de phytostérol,
- les polyesters non réticulés résultant de la polycondensation entre un diacide ou un polyacide carboxylique linéaire ou ramifié en C4-C50 et un diol ou un polyol en C2-C50, et
- les esters aliphatiques d'ester résultant de l'estérification d'un ester d'acide hydroxycarboxylique aliphatique par un acide carboxylique aliphatique,
- et leurs mélanges.

24. Composition selon la revendication 23, **caractérisée en ce que** l'acide carboxylique aliphatique de l'ester aliphatique comprend de 4 à 30 et de préférence de 6 à 30 atomes de carbone, et est de préférence choisi parmi l'acide héxanoïque, l'acide heptanoïque, l'acide octanoïque, l'acide éthyl-2 héxanoïque, l'acide nonanoïque, l'acide décanoïque, l'acide undécanoïque, acide dodécanoïque, l'acide tridécanoïque, l'acide tétradécanoïque, l'acide pentadécanoïque, l'acide héxadécanoïque, l'acide héxyldécanoïque, l'acide heptadécanoïque, l'acide octadécanoïque, l'acide isostéarique, l'acide nonadécanoïque, l'acide eicosanoïque, l'acide isoarachidique, l'acide octyldodécanoïque, l'acide henéicosanoïque, l'acide docosanoïque, et leurs mélanges.

25. Composition selon la revendication 23, **caractérisée en ce que** l'ester d'acide hydroxy carboxylique aliphatique est issu d'un acide carboxylique aliphatique hydroxylé comportant de 2 à 40 atomes de carbone, de préférence de 10 à 34 atomes de carbone et mieux de 12 à 28 atomes de carbone, et de 1 à 20 groupes hydroxyle, de préférence de 1 à 10 groupes hydroxyle et mieux de 1 à 6 groupes hydroxyle.

26. Composition selon la revendication 25, **caractérisée en ce que** l'ester d'acide hydroxy carboxylique aliphatique est choisi parmi :
a) les esters partiels ou totaux de monoacides carboxyliques aliphatiques mono hydroxylés linéaires, saturés ;
b) les esters partiels ou totaux de monoacides carboxyliques aliphatiques mono hydroxylés insaturés ;
c) les esters partiels ou totaux de polyacides carboxyliques aliphatiques mono hydroxylés saturés ;
d) les esters partiels ou totaux de polyacides carboxyliques aliphatiques poly hydroxylés saturés ;
e) les esters partiels ou totaux de polyols aliphatiques en C₂ à C₁₆ ayant réagi avec un mono ou un poly acide carboxylique aliphatique mono ou poly hydroxylé,
et leurs mélanges.

27. Composition selon la revendication 26, **caractérisée en ce que** l'ester d'acide hydroxy carboxylique aliphatique est avantageusement choisi parmi :
- l'ester résultant de la réaction d'estérification de l'huile de ricin hydrogénée avec l'acide isostéarique dans les proportions 1 pour 1 (1/1) ou monoisostéarate d'huile de ricin hydrogénée,
- l'ester résultant de la réaction d'estérification de l'huile de ricin hydrogénée avec l'acide isostéarique dans les proportions 1 pour 2 (1/2) ou le diisostéarate d'huile de ricin hydrogénée,
- l'ester résultant de la réaction d'estérification de l'huile de ricin hydrogénée avec l'acide isostéarique dans les proportions 1 pour 3 (1/3) ou triisostéarate d'huile de ricin hydrogénée,
- et leurs mélanges.

28. Composition selon l'une des revendications précédentes, **caractérisée en ce qu'**elle comprend en outre une phase huileuse comprenant au moins 70% en poids d'une huile de masse molaire comprise entre 650 et 10.000 g/mol, de préférence entre 750 et 7.500 g/mol.

29. Composition selon la revendication 28, **caractérisée en ce que** l'huile de masse molaire élevée est choisie parmi :
- les polymères lipophiles
- les esters d'acides gras linéaires ayant un nombre total de carbones allant de 35 à 70
- les esters hydroxylés
- les esters aromatiques
- les esters d'alcool gras ou d'acides gras ramifiés en C24-C28
- les huiles siliconées
- les huiles d'origine végétale
et leurs mélanges.

30. Composition selon la revendication 28 ou 29, **caractérisée en ce que** l'huile de masse molaire élevée est choisie parmi les polybutylènes, les polyisobutylènes hydrogénés, les polydécènes, les polydécènes hydrogénés, les copolymères de la vinylpyrrolidone tel que le PVP / héxadécène copolymère, le tétrapélargonate de pentaérythrityle, le triisostéarate de polyglycérol-2, le tridécyl trimellitate, le citrate de triisoarachidyle, le tétraisononanoate de pentaérythrityle, le triisostéarate de pentaérythrityle, le tétraisostéarate de polyglycéryle-2, le tétra décyl-2 tétradécanoate de pentaérythrityle, les silicones phénylées, l'huile de sésame, et leurs mélanges.

31. Composition selon la revendication 28, **caractérisée en ce qu'**elle comprend une phase huileuse comprenant au moins 80%, de préférence 85% en poids d'une huile de masse molaire comprise entre 650 et 10.000 g/mol, de préférence entre 750 et 7.500 g/mol.

32. Composition selon l'une des revendications précédentes, se présentant sous forme de produit de maquillage du corps, d'un rouge ou brillant à lèvres, d'un mascara, d'un vernis à ongles, d'un produit de coloration ou de soin des cheveux, d'un déodorant.

33. Composition selon l'une des revendications précédentes, **caractérisée en ce qu'**elle contient, en outre, au moins un corps gras additionnel choisi parmi les huiles, les cires, les gommes, les résines, les polymères lipophiles et leurs mélanges.

34. Composition selon l'une des revendications précédentes, **caractérisée en ce qu'**elle contient au moins une matière colorante.

35. Composition selon la revendication précédente, **caractérisée en ce que** la matière colorante est choisie parmi les colorants solubles ou dispersibles dans la composition, les pigments, les nacres et leurs mélanges.

36. Composition selon l'une des revendications précédentes, **caractérisée en ce qu'**elle contient, en outre, un aditif choisi parmi les antioxydants, les actifs cosmétiques ou dermatologiques, les conservateurs, les gélifiants de corps gras liquides, les dispersants et leurs mélanges.

37. Composition selon l'une des revendications précédentes, **caractérisée en ce qu'**elle contient un actif cosmétique choisi parmi les vitamines A, E, C, B₃, les provitamines comme le D-panthénol, les actifs apaisants comme l'α-bisabolol l'aloe vera, l'allantoïne, les extraits de plantes ou les huiles essentielles, les agents protecteurs ou restructurants comme les céramides, les actifs fraîcheur comme le menthol et ses dérivés, les émollients (beurre de cacao, diméthicone), les hydratants (arginine PCA), les actifs antirides, les acides gras essentiels, et leurs mélanges.

38. Composition selon l'une des revendications précédentes, **caractérisée en ce qu'**elle se présente sous forme coulée ou compactée.

39. Composition selon l'une des revendications précédentes, **caractérisée en ce qu'**elle se présente sous forme d'une phase continue huileuse.

40. Composition selon l'une des revendications précédentes, **caractérisée en ce qu'**elle se présente sous forme d'un rouge à lèvres.

41. Utilisation (i) d'au moins un ester à groupement aromatique, liquide à température ambiante, résultant de l'estérification par un acide aromatique d'au moins un groupe hydroxyle pendant ou en bout de chaîne d'un composé aliphatique hydroxylé choisi parmi les acides aliphatiques hydroxylés et leurs esters, et (ii) d'au moins un composé pâteux différent de la lanoline ayant une dureté à 25°C comprise entre 0,001 à 0,5 MPa, de préférence de 0,002 à 0,4 MPa, et dont la fraction liquide à 23°C est comprise entre 9 et 97% en poids, de préférence entre 15 et 85%, de préférence encore entre 40 et 85% en poids .
dans une composition
pour conférer à un film de ladite composition des propriétés de brillance, de confort, de tenue dans le temps, de non collant, non gras, de bon étalement et/ou de glissant, et/ou pour limiter l'exsudation de ladite composition.
